(19) Europäisches Patentamt
European Patent Office
Office européen des brevets

(11) Veröffentlichungsnummer: **0 191 723**
**A1**

(12) # EUROPÄISCHE PATENTANMELDUNG

(21) Anmeldenummer: 86730022.0

(22) Anmeldetag: 13.02.86

(51) Int. Cl.⁴: **C07C 43/29**, C07C 143/68, C07C 147/06, C07D 213/64, A01N 31/14, A01N 41/02, A01N 43/40

Ein Antrag gemäss Regel 88 EPü auf Hinzufügung der Seite 6, Beschreibung liegt vor. über diesen Antrag wird im Laufe des Verfahrens von der Prüfungsabteilung eine Entscheidung getroffen werden (Richtlinien für die Prüfung im EPA, A-V, 2.2).

(30) Priorität: 14.02.85 DE 3505370

(43) Veröffentlichungstag der Anmeldung:
**20.08.86 Patentblatt 86/34**

(84) Benannte Vertragsstaaten:
**AT BE CH DE FR GB IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Franke, Helga, Dr.**
**Karl-Stieler-Strasse 2b**
**D-1000 Berlin 41(DE)**
Erfinder: **Franke, Heinrich, Dr.**
**Fabeckstrasse 38**
**D-1000 Berlin 33(DE)**
Erfinder: **Krüger, Hans-Rudolf, Dr.**
**Kulmbacherstrasse 15**
**D-1000 Berlin 30(DE)**
Erfinder: **Joppien, Hartmut, Dr.**
**Juttastrasse 18**
**D-1000 Berlin 37(DE)**

(54) **Aromatische Alkanderivate, Schädlingsbekämpfungsmittel enthaltend diese Vebindungen sowie Verfahren zu ihrer Herstellung.**

(57) Die Erfindung betrifft aromatische Alkanderivate der allgemeinen Formel

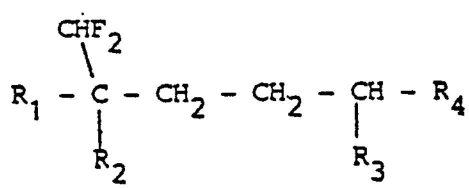

in der

$R_1$ Aryl, durch $C_1$-$C_4$-Alkyl, Halogen-$C_1$-$C_4$-alkyl, Phenyl-$C_1$-$C_4$-alkyl, $C_2$-$C_4$-Alkenyl, Halogen-$C_2$-$C_4$-alkenyl, Phenyl-$C_2$-$C_4$-alkenyl, $C_2$-$C_4$-Alkinyl, Halogen-$C_2$-$C_4$-alkinyl, Phenyl-$C_2$-$C_4$alkinyl, $C_1$-$C_4$-Alkoxy, Halogen-$C_1$-$C_4$-alkoxy, Phenyl-$C_1$-$C_4$-alkoxy, $C_2$-$C_4$-Alkenyloxy, Halogen-$C_2$-$C_4$-alkenyloxy, Phenyl-$C_2$-$C_4$-alkenyloxy, $C_2$-$C_4$-Alkinyloxy, Halogen-$C_2$-$C_4$-alkinyloxy, Phenyl-$C_2$-$C_4$-alkinyloxy, Alkylsulfonyloxy, Halogenalkylsulfonyloxy, Arylsulfonyloxy, Halogen, Cyan, Nitro, Aryloxy, Halogenaryloxy, $C_1$-$C_4$-Alkyl-aryloxy, oder Nitroaryloxy substituiertes Aryl,

$R_2$ Wasserstoff oder $C_1$-$C_4$-Alkyl,

$R_3$ Wasserstoff, Cyano oder Ethinyl,

$R_4$ Phenyl, Pyridyl oder durch $C_1$-$C_6$-Alkyl, Halogen-$C_1$-$C_6$-alkyl, Phenyl-$C_1$-$C_6$-alkyl, durch O-, N- oder S-Atome unterbrochenes $C_1$-$C_6$-Alkyl, $C_2$-$C_4$-Alkenyl, Halogen-$C_2$-$C_4$-alkenyl, Phenyl-$C_2$-$C_4$-alkenyl, $C_1$-$C_4$-Alkoxy, Halogen-$C_1$-$C_4$-alkoxy, Phenyl-$C_1$-$C_4$-alkoxy, $C_2$-$C_4$-Alkenyloxy, Halogen-$C_2$-$C_4$-alkenyloxy, Phenyl-$C_2$-$C_4$-alkenyloxy, $C_2$-$C_4$-Alkinyloxy, Halogen-$C_2$-$C_4$-alkinyloxy, Phenyl-$C_2$-$C_4$-alkinyloxy, Aryloxy, Halogenaryloxy, $C_1$-$C_4$-Alkylaryloxy, Arylamino, Halogenarylamino, $C_1$-$C_4$-Alkylarylamino, Aryl-N-$C_1$-$C_4$-alkyl-amino, Aryl-N-$C_1$-$C_4$-acyl-amino, Aroyl, Halogenaroyl, $C_1$-$C_4$-Alkylaroyl, Aryl, Halogenaryl, $C_1$-$C_4$-Alkylaryl oder durch Halogen einoder mehrfach substituiertes Phenyl oder Pyridyl

EP 0 191 723 A1

darstellen, und Schädlingsbekämpfungsmittel enthaltend diese Verbindungen sowie Verfahren zu ihrer Herstellung.

# Aromatische Alkanderivate, Schlädingsbekämpfungsmittel enthaltend diese Verbindungen sowie Verfahren zu ihrer Herstellung

Die Erfindung betrifft neue aromatische Alkanderivate, Schädlingsbekämpfungsmittel enthaltend diese Verbindungen sowie Verfahren zu ihrer Herstellung.

Verbindungen gleicher Wirkungsrichtung sind bereits bekannt (DE-PS 1 493 646; DE-PS 1 076 662; CH-PS 226 180).

Aufgabe der vorliegenden Erfindung ist die Zurverfügungstellung von neuen Verbindungen, die Schadinsekten und Milben wesentlich besser bekämpfen als die für diesen Zweck bekannten Verbindungen.

Es wurde nun gefunden, daß Verbindungen der allgemeinen Formel

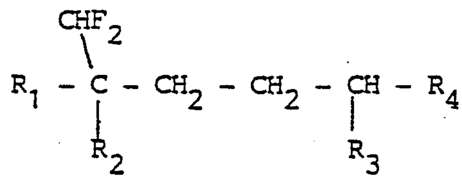

in der

$R_1$ Aryl, durch $C_1$-$C_4$Alkyl, Halogen-$C_1$-$C_4$-alkyl, Phenyl-$C_1$-$C_4$-alkyl, $C_2$-$C_4$-Alkenyl, Halogen-$C_2$-$C_4$-alkenyl, Phenyl-$C_2$-$C_4$-alkenyl, $C_2$-$C_4$-Alkinyl, Halogen-$C_2$-$C_4$-alkinyl, Phenyl-$C_2$-$C_4$alkinyl, $C_1$-$C_4$-Alkoxy, Halogen-$C_1$-$C_4$-alkoxy, Phenyl-$C_1$-$C_4$-alkoxy, $C_2$-$C_4$-Alkenyloxy, Halogen-$C_2$-$C_4$-alkenyloxy, Phenyl-$C_2$-$C_4$-alkenyloxy, $C_2$-$C_4$-Alkinyloxy, Halogen-$C_2$-$C_4$-alkinyloxy, Phenyl-$C_2$-$C_4$-alkinyloxy, Alkysulfonyloxy, Halogenalkylsulfonyloxy, Arylsulfonyloxy, Halogen, Cyan, Nitro, Aryloxy, Halogenaryloxy, $C_1$-$C_4$-Alkyl-aryloxy oder Nitroaryloxy substituiertes Aryl,

$R_2$ Wasserstoff oder $C_1$-$C_4$-Alkyl,

$R_3$ Wasserstoff, Cyano oder Ethinyl,

$R_4$ Phenyl, Pyridyl oder durch $C_1$-$C_6$-Alkyl, Halogen-$C_1$-$C_6$-alkyl, Phenyl-$C_1$-$C_6$-alkyl, durch O-, N- oder S-Atome unterbrochenes $C_1$-$C_6$-Alkyl, $C_2$-$C_4$-Alkenyl, Halogen-$C_2$-$C_4$-alkenyl, Phenyl-$C_2$-$C_4$-alkenyl, $C_1$-$C_4$-Alkoxy, Halogen-$C_1$-$C_4$-alkoxy, Phenyl-$C_1$-$C_4$-alkoxy, $C_2$-$C_4$- Alkenyloxy, Halogen-$C_2$-$C_4$-alkenyloxy, Phenyl-$C_2$-$C_4$-alkenyloxy, $C_2$-$C_4$-Alkinyloxy, Halogen-$C_2$-$C_4$-alkinyloxy, Phenyl-$C_2$-$C_4$-alkinyloxy, Aryloxy, Halogenaryloxy, $C_1$-$C_4$-Alkylaryloxy, Arylamino, Halogenarylamino, $C_1$-$C_4$-Alkylarylamino, Aryl-N-$C_1$-$C_4$-alkyl-amino, Aryl-N-$C_1$-$C_4$-acyl-amino, Aroyl, Halogenaroyl, $C_1$-$C_4$-Alkylaroyl, Aryl, Halogenaryl, $C_1$-$C_4$-Alkylaryl oder durch Halogen einoder mehrfach substituiertes Phenyl oder Pyridyl

bedeuten, bekannte wichtige Schadinsekten und Milben überraschenderweise wirksamer bekämpfen als bekannte Verbindungen gleicher Wirkungsrichtung.

Der in der allgemeinen Formel I als $R_1$ bezeichnete Arylrest umfaßt auch die Reste 1-Naphthyl, 2-Naphthyl, Benzofuran-5-yl, Benzothiophen-5-yl, Benzofuran-6-yl, Benzothiophen-6-yl, Benzoxazol-5-yl, Benzoxazol-6-yl, Indan-5-yl, Indan-6-yl, 1,4-Benzodioxan-6-yl, 1,3-Benzodioxan-6-yl 2,3-Benzodioxan-7-yl und 1,3-Benzodioxol-5-yl.

Von den erfindungsgemäßen Verbindungen zeichnen sich insbesondere diejenigen aus, bei denen in der allgemeinen Formel I

$R_1$ Chlorphenyl, Bromphenyl, Fluorphenyl, Methylphenyl, Methoxyphenyl, Ethoxyphenyl, Difluormethoxyphenyl, Fluorethoxyphenyl, Trifluorethoxyphenyl,

$R_2$ Methyl,

$R_3$ Wasserstoff und

$R_4$ Phenoxyphenyl, Fluor-phenoxyphenyl oder Phenoxypyridyl darstellen.

Die erfindungsgemäßen Verbindungen treten als optische Isomere auf. Die einzelnen Isomeren und ihre Mischungen gehören auch zum Gegenstand der Erfindung.

Die Anwendung der erfindungsgemäßen Verbindungen kann in Konzentrationen von 0,0005 bis 5,0 %, vorzugsweise von 0,001 bis 0,1 % erfolgen.

Die erfindungsgemäßen Verbindungen können entweder allein, in Mischung miteinander oder mit anderen insektiziden Wirkstoffen angewendet werden. Gegebenenfalls können andere Pflanzenschutzmittel oder Schädlingsbekämpfungsmittel wie zum Beispiel Akarizide oder Fungizide je nach dem gewünschten Zweck zugesetzt werden.

Eine Förderung der Wirkungsintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls von Netz- Haft-, Emulgier-und/oder Dispergierhilfsmitteln angewandt.

Geeignete flüssige Trägerstoffe sind zum Beispiel Wasser, aliphatische und aromatische Kohlenwasserstoffe, weiterhin Cyclohexanon, Isophoron, Dimethylsulfoxid, Dimethylformamid und Mineralölfraktionen.

Als feste Trägerstoffe eignen sich Mineralerden, zum Beispiel Tonsil, Silikagel, Talkum, Kaolin, Attapulgit, Kalkstein, Kieselsäure und pflanzliche Produkte, zum Beispiel Mehle.

An oberflächenaktiven Stoffen sind zum Beispiel zu nennen: Calciumligninsulfonat, Polyethylen-alkylphenylether, Naphthalinsulfonsäuren und deren Salze, Phenolsulfonsäuren und daren Salze, Formaldehydkondensate, Fettalkoholsulfate sowie substituierte Benzolsulfonsäuren und deren Salze.

Der Anteil der bzw. des Wirkstoffe(s) in den verschiedenen Zubereitungen kann in weiten Grenzen variieren. Beispielsweise enthalten die Mittel etwa 5 bis 95 Gewichtsprozent Wirkstoffe, etwa 95 bis 5 Gewichtsprozent flüssige oder feste Trägerstoffe sowie gegebenenfalls bis zu 20 Gewichtsprozente oberflächenaktive Stoffe.

Die Ausbringung der Mittel kann in üblicher Weise erfolgen, zum Beispiel mit Wasser als Träger in Spritzbrühmengen von etwa 100 bis 3000 Liter/ha. Eine Anwendung der Mittel im sogenannten Low-Volume- und Ultra-Low-Volume-Verfahren ist ebenso möglich wie ihre Applikation in Form von sogenannten Mikrogranulaten.

Die Herstellung dieser Zubereitungen kann in an sich bekannter Art und Weise zum Beispiel durch Mahl- oder Mischverfahren, durchgeführt werden. Gewünschtenfalls können die Einzelkomponenten auch erst kurz vor ihrer Verwendung gemischt werden, wie es zum Beispiel im sogenannten Tankmixverfahren in der Praxis durchgeführt wird.

Zur Herstellung der Zubereitungen werden zum Beispiel die folgenden Bestandteile eingesetzt:

A) 20 Gewichtsprozent Wirkstoff

35 Gewichtsprozent Bentonit

8 Gewichtsprozent Calciumsalz der Ligninsulfonsäure

2 Gewichtsprozent Natriumsalz des N-Methyl-N-oleyltaurins

35 Gewichtsprozent Kieselsäure

B) 45 Gewichtsprozent Wirkstoff

5 Gewichtsprozent Natriumaluminiumsilikat

15 Gewichtsprozent Cetylpolyglykolether mit 8 Mol Ethylenoxid

2 Gewichtsprozent Spindelöl

10 Gewichtsprozent Polyethylenglycol

23 Teile Wasser

C) 20 Gewichtsprozent Wirkstoff

75 Gewichtsprozent Isophoron

5 Gewichtsprozent Kombinationsemulgator aus Calciumphenylsulfonat und Fettalkoholpolyglycolether

D) 80 Gewichtsprozent Wirkstoff

15 Gewichtsprozent Kaolin

5 Gewichtsprozent oberflächenaktive Stoffe auf Basis des Natriumsalzes des N-Methyl-N-oleyl-taurins und des Calciumsalzes der Liginsulfonsäure

Die erfindugnsgemäßen neuen Verbindungen lassen sich herstellen, indem man

a) Verbindungen der allgemeinen Formel

$$R_5\overset{\oplus}{P} - CH_2\ CH_2R_4X^{\ominus} \qquad II$$

oder

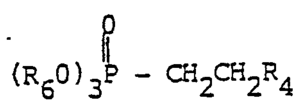

V

zunächst mit einer Base und darauf mit Verbindungen der allgemeinen Formel

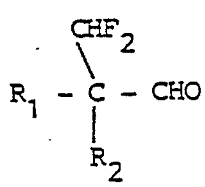

III

unter Bildung von Verbindungen der allgemeinen Formel

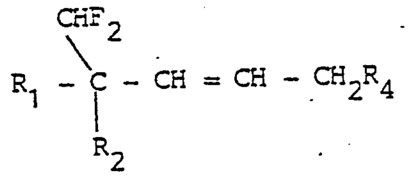

IV

und diese zu den gewünschten Verfahrensprodukten reduziert oder

b) Verbindungen der allgemeinen Formel

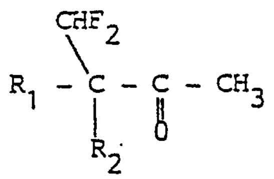

VI

mit Aldehyden der allgemeinen Formel

$R_4$ - CHO VII

oder aromatische Kohlenwasserstoffe, wie zum Beispiel Hexan, Benzol oder Toluol, und Ether, wie zum Beispiel Diethylether, Tetrahydrofuran. Geeignet sind ferner Amide, wie Dimethylformamid oder Hexamethylphosphorsäuretriamid, außerdem können Alkohole oder auch Dimethylsulfoxid verwendet werden.

Als für die Reaktionsvariante a) geeignete Basen können Metallalkoholate, wie zum Beispiel Natriumethanolat, Metallhydride, wie zum Beispiel Natriumhydrid, Metallamide, wie zum Beispiel Natriumamid und Organometallverbindungen, wie zum Beispiel Phenyllithium oder Butyllithium, eingesetzt werden.

Verbindungen der allgemeinen Formel I, in denen der Rest $R_1$ eine Alkoxyoder Halogenalkoxyphenylgruppe usw. ist, können auch durch Umsetzen eines Hydroxyphenylderivats das durch Hydrolyse eines anderen Alkoxyphenylderivats hergestellt werden kann, mit der entsprechenden halogenierten Verbindung erhalten werden.

Die als Ausgangsstoffe für die Reaktionsvariante a) zu verwendenden Verbindungen der allgemeinen Formel II oder V können leicht durch Umsetzen von $R_4CH_2CH_2X$, worin X für ein Halogenatom steht, mit $(R_5)_3P$ oder $(R_60)_3P$ erhalten werden.

Die als Ausgangsstoffe zu verwendenden Aldehyde der allgemeinen Formel III lassen sich zum Beispiel durch Reduktion der entsprechenden Nitrile darstellen. Die Reduktion erfolgt nach an sich bekannten Methoden mit Alkylaluminiumhydriden, wie zum Beispiel Diisobutylaluminiumhydrid. Die erforderlichen Nitrile lassen sich durch Alkylieren eines entsprechenden Arylacetonitrils mit $CHClF_2$ herstellen.

Die Verbindungen mit $R_3$ = Cyano werden zum Beispiel aus den entsprechenden Ausgangsverbindungen hergestellt, bei denen $R_3$ Wasserstoff darstellt, welche mit N-Bromsuccinimid bromiert werden und man das Brom anschließend gegen Cyanid austauscht.

Die folgenden Beispiele erläutern die Herstellung der erfindungsgemäßen Verbindungen.

## BEISPIEL 1

2-(4-Ethoxyphenyl)-1,1-difluor-5-(4-fluor-3-phenoxyphenyl)-2-methylpentan

2,0 g (4,69 mmol) 4-(4-Ethoxyphenyl)-5,5-difluor-1-(4-fluor-3-phenoxyphenyl)-4-methyl-2-penten werden in 30 ml Ethanol mit Wasserstoff unter Zusatz von 0,2 g Raney-Ni bei Raumtemperatur unter Atmosphärendruck hydriert. Nach Aufnahme der berechneten Wasserstoffmenge wird der Katalysator abfiltriert und das Lösungsmittel unter vermindertem Druck abgezogen. Nach Chromatographie mit Hexan/Toluol an Kieselgel verbleiben 1,8 g des gewünschten Produkts. Das sind 89,6 % der Theorie.

Brechungsindex $n_D20$ : 1,5481

| | | | | |
|---|---|---|---|---|
| Analyse: | Berechnet | C 72,88 % | H 6,35 % | F 13,30 % |
| | Gefunden | C 72,93 % | H 6,31 % | F 13,31 % |

Die erforderlichen Ausgangs- bzw. Zwischenprodukte lassen sich wie folgt herstellen:

4-(4-Ethoxyphenyl)-5,5-difluor-1-(4-fluor-3-phenoxyphenyl)-4-methyl-2-penten

Zu 6,38 g (11,4 mmol) (2-(4-Fluor-3-phenoxyphenyl)-ethyl)-triphenyl-phosphoniumbromid in 40 ml absolutem Tetrahydrofuran werden bei Raumtemperatur unter $N_2$-Atmosphäre innerhalb von 5 Minuten 7,15 ml einer 1,6molaren Butyllithium-Lösung in n-Hexan zugetropft. Nach einstündigem Rühren wird eine Lösung aus 2,58 g - (11,3mmol) 2-Difluormethyl-2-(4-ethoxyphenyl)-propionaldehyd in 10 ml absolutem Tetrahydrofuran zugetropft. Es wird 18 Stunden bei Raumtemperatur gerührt. Man gibt auf Eiswasser, extrahiert mit Ether, trocknet mit Natriumsulfat und dampft ein. Nach Chromatographie mit Hexan/Toluol an Kieselgel verbleiben 2,07 g 4-(4-Ethoxyphenyl)-5,5-difluor-1-(4-fluor-3-phenoxyphenyl)-4-methyl-2-penten, das sind 43% der Theorie.

Brechungsindex: $n_D20$ : 1,5549

2-Difluormethyl-2-(4-ethoxyphenyl)-propionaldehyd

Zu 7,5 g (33,3 mmol) 2-Difluormethyl-2-(4-ethoxyphenyl)-propionitril in 75 ml Tuluol werden unter Eiskühlung und $N_2$-Atmosphäre 30,5 ml einer 1,2 molaren Diisobutylaluminiumhydrid-Lösung in Toluol getropft. Anschließend wird 1,5 Stunden bei Raumtemperatur gerührt. Man hydrolysiert die Reaktionsmischung mit 10 %iger Schwefelsäure, extrahiert mit Essigester, trocknet mit Natriumsulfat und zieht das Lösungsmittel unter vermindertem Druck ab.

Es verbleiben 6,47 g des gewünschten Produktes als hellgelbes Öl, das sind 93,1 % der Theorie

DC: Hexan/Ether 1:1 RF: 0,57

BEISPIEL 1 A

2-(4-Ethoxyphenyl)-1,1-difluor-5-(4-fluor-3-phenoxyphenyl)-2-methylpentan

1,5 g (3,75 mmol) 1,1-Difluor-5-(4-fluor-3-phenoxyphenyl)-2-(4-hydroxyphenyl)-2-methylpentan, 10 ml Dimethylformamid, 700 mg (4.5 mmol) Ethyljodid und 725 mg (5,25 mmol) $K_2CO_3$ werden mit einer katalytischen Menge Natriumjodid 8 Stunden auf 80°C erwärmt. Anschließend wurde in Wasser gegeben, mit Essigester extrahiert, mit $Na_2SO_4$ getrocknet und eingeengt. Nach Chromatographie mit Hexan/Toluol an Kieselgel verbleiben 1,4 g des gewünschten Produktes, das sind 87,4 % der Theorie. Die physikalischen Daten sind mit denen des nach Beispiel 1 dargestellten Produktes identisch.

Das erforderliche Ausgangsprodukt läßt sich wie folgt herstellen.

1,1-Difluor-5-(4-fluor-3-phenoxyphenyl)-2-(4-hydroxyphenyl)-2-methylpentan

27,22 g (65,68 mmol) 1,1-Difluor-5-(4-fluor-3-phenoxyphenyl)-2-(4-methoxyphenyl)-2-methylpentan werden mit 75,92 g (657 mmol) Pyridinhydrochlorid 7 Stunden auf 180°C erhitzt. Anschließend wird in Wasser aufgenommen, mit Essigester extrahiert und die organische Phase neutral gewaschen, mit $Na_2SO_4$ getrocknet und eingeengt. Nach Chromatographie mit Toluol/Essigester an Kieselgel verbleiben 24,5 g des gewünschten Produktes, das sind 93,1 % der Theorie.

Brechungsindex $n_D20$ : 1,5571

In analoger Weise werden die weiteren erfindungsgemäßen Verbindungen hergestellt.

| Beispiel Nr. | Name der Verbindungen | Physikalische Konstante |
|---|---|---|
| 2 | 2-(4-Chlorphenyl)-1,1-difluor-2-methyl-5-(3-phenoxyphenyl)-pentan | $n_D^{20}$ 1,5689 |
| 3 | 1,1-Difluor-2-(4-methoxyphenyl)-2-methyl-5-(3-phenoxyphenyl)-pentan | $n_D^{20}$ 1,5625 |
| 4 | 1,1-Difluor-5-(4-fluor-3-phenoxy-phenyl)-2-methyl-2-(4-methyl-phenyl)-pentan | $n_D^{20}$ 1,5498 |
| 5 | 1,1-Difluor-2-methyl-2-(2-naphthyl)-5-(3-phenoxyphenyl)-pentan | $n_D^{20}$ 1,5899 |
| 6 | 2-(4-Butoxyphenyl)-1,1-difluor-2-methyl-5-(3-phenoxyphenyl)-pentan | $n_D^{20}$ 1,5474 |
| 7 | 1,1-Difluor-2-methyl-2-(4-n-propoxyphenyl)-5-(3-phenoxyphenyl)-pentan | $n_D^{20}$ 1,5526 |
| 8 | 1,1-Difluor-2-(4-isopropoxyphenyl)-2-methyl-5-(3-phenoxyphenyl)-pentan | $n_D^{20}$ 1,5517 |
| 9 | 1,1-Difluor-2-methyl-2-(4-methylsulfonyl-oxyphenyl)-5-(3-phenoxyphenyl)-pentan | $n_D^{20}$ 1,5573 |
| 10 | 1,1-Difluor-2-methyl-5-(3-phenoxyphenyl)-2-(4-trifluormethylsulfonyloxyphenyl)-pentan | $n_D^{20}$ 1,5230 |
| 11 | 1,1-Difluor-2-methyl-5-(3-phenoxy-phenyl)-2-[4-(2,2,2-trifluorethoxy)-phenyl]-pentan | $n_D^{20}$ 1,5282 |
| 12 | 2-(4-Difluormethoxyphenyl)-1,1-difluor-2-methyl-5-(3-phenoxyphenyl)-pentan | $n_D^{20}$ 1,5391 |
| 13 | 1,1-Difluor-2-[4-(2-fluorethoxy)-phenyl]-2-methyl-5-(3-phenoxyphenyl)-pentan | $n_D^{20}$ 1,5541 |
| 14 | 2-(4-Allyloxyphenyl)-1,1-difluor-2-methyl-5-(3-phenoxyphenyl)-pentan | $n_D^{20}$ 1,5612 |
| 15 | 1,1-Difluor-2-methyl-5-(3-phenoxy-phenyl)-2-(4-propargyloxyphenyl)-pentan | $n_D^{20}$ 1,5643 |

| Beispiel Nr. | Name der Verbindungen | Physikalische Konstante |
|---|---|---|
| 16 | 2-(4-tert.-Butylphenyl)-1,1-difluor--2-methyl-5-(3-phenoxyphenyl)-pentan | $n_D^{20}$ 1,5502 |
| 17 | 2-(4-Chlorphenyl)-1,1-difluor-5--(4-fluor-3-phenoxyphenyl)-2-methyl-pentan | |
| 18 | 1,1-Difluor-5-(4-fluor-3-phenoxyphenyl)-2-(4-methoxyphenyl)-2-methyl--pentan | $n_D^{20}$ 1,5530 |
| 19 | 2-(4-Butoxyphenyl)-1,1-difluor-5-(4-fluor-3-phenoxyphenyl)-2-methylpentan | $n_D^{20}$ 1,5414 |
| 20 | 1,1-Difluor-5-(4-fluor-3-phenoxyphenyl)--2-methyl-2-(4-n-propoxyphenyl)-pentan | $n_D^{20}$ 1,5453 |
| 21 | 1,1-Difluor-5-(4-fluor-3-phenoxyphenyl)--2-(4-isopropoxyphenyl)-2-methylpentan | $n_D^{20}$ 1,5442 |
| 22 | 1,1-Difluor-5-(4-fluor-3-phenoxyphenyl)-2-methyl-2-(4-methylsulfonylphenyl)-pentan | $n_D^{20}$ 1,5501 |
| 23 | 1,1-Difluor-5-(4-fluor-3-phenoxyphenyl)-2-methyl-2-(4-trifluormethylsulfonyloxyphenyl)-pentan | $n_D^{20}$ 1,5172 |
| 24 | 1,1-Difluor-5-(4-fluor-3-phenoxy-phenyl)-2-methyl-2-[4-(2,2,2-trifluor-ethoxy)-phenyl]-pentan | $n_D^{20}$ 1,5240 |
| 25 | 2-(4-Difluormethoxyphenyl)-1,1-difluor--5-(4-fluor-3-phenoxyphenyl)-2-methyl-pentan | $n_D^{20}$ 1,5323 |
| 26 | 1,1-Difluor-2-[4-(2-fluorethoxy)-phenyl]--5-(4-fluor-3-phenoxyphenyl)-2-methyl-pentan | $n_D^{20}$ 1,5469 |
| 27 | 2-(4-Allyloxyphenyl)-1,1-difluor-5--(4-fluor-3-phenoxyphenyl)-2-methyl-pentan | $n_D^{20}$ 1,5540 |
| 28 | 1,1-Difluor-5-(4-fluor-3-phenoxyphenyl)--2-methyl-2-(4-propargyloxyphenyl)-pentan | $n_D^{20}$ 1,5565 |
| 29 | 2-(4-tert.-Butylphenyl)-1,1-difluor-5--(4-fluor-3-phenoxyphenyl)-2-methylpentan | |
| 30 | 2-(4-Ethoxyphenyl)-1,1-difluor-2-methyl--5-(6-phenoxy-2-pyridyl)-pentan | |

| Beispiel Nr. | Name der Verbindungen | Physikalische Konstante |
|---|---|---|
| 31 | 2-(4-Chlorphenyl)-1,1-difluor-2-methyl-5-(6-phenoxy-2-pyridyl)-pentan | |
| 32 | 2-(4-Bromphenyl)-1,1-difluor-5-(4-fluor-3-phenoxyphenyl)-2-methylpentan | |
| 33 | 1,1-Difluor-2-(4-fluorphenyl)-5-(4-fluor-3-phenoxyphenyl)-2-methylpentan | $n_D^{20}$ 1,5638 |
| 34 | 2-(4-Ethoxyphenyl)-1,1-difluor-2-methyl-5-(3-phenoxyphenyl)-pentan | $n_D^{20}$ 1,5570 |
| 35 | 2-/4-(2,2-Dichlorvinyloxy)-phenyl7-1,1-difluor-5-(4-fluor-3-phenoxyphenyl)-2-methylpentan | $n_D^{20}$ 1,5638 |

Die Verbindungen sind farb- und geruchlose Öle, die in praktisch allen organischen Lösungsmitteln gut löslich, in Wasser dagegen sehr schwer löslich sind.

Die folgenden Beispiele dienen zur Erläuterung der Anwendungsmöglichkeiten der erfindungsgemäßen Verbindungen, die in Form ihrer Zubereitungen erfolgte.

BEISPIEL 36

Abtötende Wirkung auf Larven (L3) des Mexikanischen Bohnenkäfers (Epilachna varivestis)

Die erfindungsgemäßen Verbindungen wurden als wäßrige Emulsionen mit der Wirkstoffkonzentration 0,1 % eingesetzt. In diese Wirkstoffzubereitung wurden Buschbohnenpflanzen (Phaseolus vulgaris) im Primärblattstadium getaucht. Pro Versuchsglied wurden zwei Pflanzenstengel mit insgesamt 4 Primärblättern in mit Wasser gefüllte Glasvasen eingestellt und in Glaszylindern eingekäfigt. Danach wurden je 5 Larven des Mexikanischen Bohnenkäfers (Epilachna varivestis) im 3. Larvenstadium in die Glaszylinder eingezählt und für 3 Tage darin gehalten.

Kriterium für die Wirkungsbeurteilung war die Sterblichkeit der Larven nach drei Tagen.

Es zeigte sich, daß die Verbindungen gemäß den Beispielen 1-16, 18-28 und 33-35 bei der verwendeten Wirkstoffkonzentration eine 100 %ige Mortalität bewirkten.

BEISPIEL 37

Abtötende Wirkung auf Junglarven der Kohlschabe - (Plutella xylostella)

Die erfindungsgemäßen Verbindungen sowie die Vergleichssubstanzen wurden als wäßrige Emulsion mit der Wirstoffkonzentration 0,0025 % eingesetzt.

Mit diesen Wirkstoffzubereitungen wurden Blumenkohlblättchen in Polystyrol-Petrischalen dosiert (4 mg Spritzbrühe/cm²) gespritzt. Nach dem Antrocknen der Spritzbeläge wurden in jede Petrischale 10 Jungraupen der Kohlschabe (Plutella xylostella) eingezählt und für 2 Tage in den geschlossenen Petrischalen dem behandelten Futter exponiert.

Kriterium für die Wirkungsbeurteilung war die Sterblichkeit der Raupen in % nach 2 Tagen. Die Ergebnisse sind in nachstehender Tabelle zusammengefaßt.

| Erfindungsgemäße Verbindugnen | Wirkstoffkonzentration in % | Wirkung in % |
|---|---|---|
| 2-(4-Chlorphenyl)-1,1-difluor-2-methyl-5-(3-phenoxyphenyl)-pentan | 0,0025 | 100 |
| 2-(4-Ethoxyphenyl)-1,1-difluor-5-(4-fluor-3-phenoxyphenyl)-2-methylpentan | 0,0025 | 100 |
| 1,1-Difluor-5-(4-fluor-3-phenoxyphenyl)-2-methyl-2-(4-n-propoxyphenyl)-pentan | 0,0025 | 60 |
| 1,1-Difluor-5-(4-fluor-3-phenoxyphenyl-2-(4-isopropoxyphenyl)-2-methylpentan | 0,0025 | 85 |
| VERGLEICHSMITTEL (gemäß DE-PS 149 36 46) | | |
| 2,3-Dihydro-2,2-dimethylbenzofuran-7-ylmethylcarbamat | 0,0025 | 20 |
| VERGLEICHSMITTEL (gemäß DE-PS 107 66 62) | | |
| 0,0-Dimethyl-S-methylcarbamoylmethyl-phosphorodioat | 0,0025 | 0 |
| VERGLEICHSMITTEL (gemäß CH-PS 226 180) | | |
| 1,1,1-Trichloro-2,2-bis(4-methoxyphenyl)-ethan | 0,0025 | 0 |

BEISPIEL 38

Abtötende Wirkung auf Larven (L2) des Ägyptischen Baumwollwurms (Spodoptera littoralis)

Die erfindungsgemäßen Verbindungen sowie die Vergleichssubstanzen wurden als wäßrige Emulsionen mit der Wirkungskonzentration 0,016 % eingesetzt. Mit diesen Wirkstoffzubereitungen wurden je ein Fiederblattpaar der Puffbohne (Vicia faba) sowie 10 Larven (L2) des Ägyptischen Baumwollwurms (Spodoptera littoralis) pro Versuchsglied mit 4 mg Spritzbrühe/cm² in Polystyrol-Petrischalen dosiert gespritzt. Die geschlossenen Petrischalen wurden dann im Labor unter Langtagbedingungen für 2 Tage aufgestellt. Kriterium für die Wirkungsbeurteilung war die Mortalität der Larven in % nach 2 Tagen.

Die nachstehende Tabelle faßt die Ergebnisse zusammen.

| Erfindungsgemäße Verbindungen | Wirkstoffkonzentration in % | Wirkung in % |
|---|---|---|
| 1,1-Difluor-5-(4-fluor-3-phenoxyphenyl)-2-methyl-2-(4-methylphenyl)-pentan | 0,016 | 100 |
| 1,1-Difluor-2-(4-isopropoxyphenyl)-2-methyl-5-(3-phenoxyphenyl)-pentan | 0,016 | 100 |
| 2-(4-Ethoxyphenyl)-1,1-difluor-5-(4-fluor-3-phenoxyphenyl)-2-methylpentan | 0,016 | 100 |
| 1,1-Difluor-5-(4-fluor-3-phenoxyphenyl)-2-methyl-2-(4-n-propoxyphenyl)-pentan | 0,016 | 90 |
| 1,1-Difluor-5-(4-fluor-3-phenoxyphenyl)-2-(4-isopropoxyphenyl)-2-methylpentan | 0,016 | 100 |
| VERGLEICHSMITTEL (gemäß DE-PS 149 36 46) | | |
| 2,3-Dihydro-2,2-dimethylbenzofuran-7-ylmethylcarbamate | 0,016 | 0 |
| VERGLEICHSMITTEL (gemäß DE-PS 107 66 62) | | |
| 0,0-Dimethyl-S-methylcarbamoylmethyl-phosphorodithioat- | 0,016 | 0 |
| VERGLEICHSMITTEL (gemäß CH-PS 226 180) | | |
| 1,1,1-Trichloro-2,2-bis(4-methoxyphenyl)-ethan- | 0,016 | 0 |

BEISPIEL 39

Abtötende Wirkung auf bewegliche Stadien und Eier der Grünen Bohnenspinnmilbe (Tetranychus urticae)

Eine Auswahl der erfindungsgemäßen Verbindungen wurden als wässrige Emulsionen mit der Wirkstoffkonzentration 0,1 % eingesetzt. Mit diesen Wirkstoffzubereitungen wurden getopfte und künstlich mit Spinnmilben (Tetranychus urticae) besetzte Buschbohnenpflanzen - (Phaseolus vulgaris) im Primärblattstadium tropfnaß gespritzt und für 7 Tage im Labor aufgestellt. Danach wurde die Mortalität der beweglichen Stadien einerseits und der Eier andererseits mit Hilfe einer vergrößernden Lupe in Prozent geschätzt.

Die folgende Tabelle zeigt die erhaltenen Ergebnisse.

| Verbindung gemäß Beispiel | Wirkstoffkonz. in % | Wirkung in % auf bewegliche Stadien/Eier von Tetranychus urticae | |
|---|---|---|---|
| 2 | 0,1 | 100 | 20 |
| 1 | 0,1 | 100 | 0 |
| 7 | 0,1 | 100 | 0 |
| 8 | 0,1 | 100 | 0 |
| 10 | 0,1 | 100 | 50 |
| 11 | 0,1 | 100 | 20 |
| 12 | 0,1 | 100 | 0 |
| 19 | 0,1 | 100 | 0 |
| 20 | 0,1 | 100 | 100 |
| 27 | 0,1 | 100 | 80 |
| 28 | 0,1 | 100 | 0 |
| 22 | 0,1 | 100 | 0 |
| 26 | 0,1 | 100 | 50 |
| 24 | 0,1 | 100 | 100 |
| 25 | 0,1 | 100 | 70 |
| 23 | 0,1 | 100 | 100 |
| 33 | 0,1 | 100 | 100 |

BEISPIEL 40

Insektizide bzw. akarizide Wirkung gegen Boophilus microplus (1), Lucilia sericata (2), Musca domestica (3) und Blattella germanica (4)

(1) Filterpapier (9 cm Ø) wird mit 1 ml eines aliquoten Teiles einer Lösung der Testsubstanz in Aceton bei verschiedenen Konzentrationen getränkt. Nach Trocknung werden die Filterpapiere zu Umschlägen gefaltet, in welche Zecken-Larven (Boophilus microplus) eingebracht und für 48 Stunden bei 25 °C und 80 % Raumfeuchte gehalten werden. Anschließend wird die prozentuale Mortalität der Larven festgestellt und mit den Kontrollversuchen verglichen.

Die Kontrollversuche ergaben eine Mortalität von <5 %, währenddessen die Verbindungen gemäß den Beispielen 1, 3, 6-8, 10-12, 14, 18-23, 25, 27, 28 und 34 eine 90 %ige Mortalität bei einer Konzentration von 100 ppm oder weniger ergaben.

(2) 1 ml eines aliquoten Teiles einer Lösung der Testsubstanz in Aceton bei verschiedenen Konzentrationen wird auf Baumwoll-Dentalrollen (1 cm x 2 cm) aufgebracht, die sich in Glasröhrchen (2 cm Ø und 5 cm Länge) befinden. Nach Trocknung wird das so behandelte Material mit 1 ml einer Nährlösung getränkt, die von Junglarven der Schafschmeißfliege (Lucilia sericata) durchsetzt ist. Dann werden die Glasröhrchen mit einem Baumwollstopfen verschlossen und für 24 Stunden bei 25 °C gehalten.

Die Auswertung der Versuche ergab, daß bei den Kontrollversuchen die Mortalität <5 % war, währenddessen die Verbindungen gemäß den Beispielen 1, 3-5, 10, 14-16, 18-25, 27, 28 und 34 eine $LC_{90}$ bei 100 ppm oder weniger aufwiesen.

(3) Aliquote Teile einer Lösung der Testsubstanz in Aceton in verschiedenen Konzentrationen werden auf Filterpapier (9 cm Ø) in Petrischalen (9 cm Ø) mit Uhrglasabdeckung aufgebracht. Nach Verdampfen des Lösungsmittels werden die so behandelten Oberflächen zusammen mit den Kontrollversuchen, die mit Aceton allein behandelt wurden, ausgewachsenen Hausfliegen (Musca domestica) exponiert und für 24 Stunden bei 22 °C gehalten. Die prozentuale Mortalität wird anschließend durch Auszählen bestimmt.

Bei den Kontrollversuchen war die Mortalität <5 %, währenddessen die Verbindungen gemäß den Beispielen 1, 16, 20, 21, 23, 25 und 34 eine $LD_{90}$ bei 100 mg/m² oder weniger aufwiesen.

(4) Aliquote Teile einer Lösung der Testsubstanz in Aceton in verschiedenen Konzentrationen werden auf Glasplatten (10 x 10 cm) aufgebracht. Nach Verdampfen des Lösungsmittels werden die so behandelten Oberflächen zusammen mit Kontrollversuchen, die mit Aceton allein behandelt wurden, mit Junglarven (L 2) die Deutschen Schabe (Blattella germanica) exponiert, indem sie durch mit Poly-Tetrafluorethylen bedampfte Glasringe (6 cm Ø) auf der behandelten Oberfläche für 24 Stunden bei 22 °C gehalten werden. Die prozentuale Mortalität der Insekten wird anschließend durch Auszählen bestimmt.

Bei den Kontrollversuchen war die Mortalität <5 %, währenddessen die Verbindungen gemäß den Beispielen 1, 6, 7, 10, 11, 12, 14, 16, 19, 20-25, 27, 28 und 34 eine $LD_{90}$ bei 100 $mg/m^2$ oder weniger zeigten.

## Ansprüche

1. Aromatische Alkanderivate der allgemeinen Formel

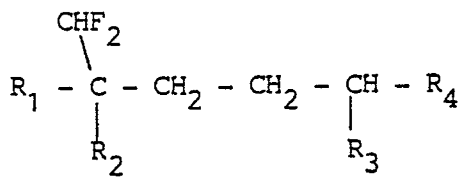

(I)

in der

$R_1$ Aryl, durch $C_1$-$C_4$Alkyl, Halogen-$C_1$-$C_4$-alkyl, Phenyl-$C_1$-$C_4$-alkyl, $C_2$-$C_4$-Alkenyl, Halogen-$C_2$-$C_4$-alkenyl, Phenyl-$C_2$-$C_4$-alkenyl, $C_2$-$C_4$-Alkinyl, Halogen-$C_2$-$C_4$-alkinyl, Phenyl-$C_2$-$C_4$alkinyl, $C_1$-$C_4$-Alkoxy, Halogen-$C_1$-$C_4$-alkoxy, Phenyl-$C_1$-$C_4$-alkoxy, $C_2$-$C_4$-Alkenyloxy, Halogen-$C_2$-$C_4$-alkenyloxy, Phenyl-$C_2$-$C_4$-alkenyloxy, $C_2$-$C_4$-Alkinyloxy, Halogen-$C_2$-$C_4$-alkinyloxy, Phenyl-$C_2$-$C_4$-alkinyloxy, Alkylsulfonyloxy, Halogenalkylsulfonyloxy, Arylsulfonyloxy, Halogen, Cyan, Nitro, Aryloxy, Halogenaryloxy, $C_1$-$C_4$-Alkyl-aryloxy oder Nitroaryloxy substituiertes Aryl,

$R_2$ Wasserstoff oder $C_1$-$C_4$-Alkyl,

$R_3$ Wasserstoff, Cyano oder Ethinyl,

$R_4$ Phenyl, Pyridyl oder durch $C_1$-$C_6$-Alkyl, Halogen-$C_1$-$C_6$-alkyl, Phenyl-$C_1$-$C_6$-alkyl, durch O-, N- oder S-Atome unterbrochenes $C_1$-$C_6$-Alkyl, $C_2$-$C_4$-Alkenyl, Halogen-$C_2$-$C_4$-alkenyl, Phenyl-$C_2$-$C_4$-alkenyl, $C_1$-$C_4$-Alkoxy, Halogen-$C_1$-$C_4$-alkoxy, Phenyl-$C_1$-$C_4$-alkoxy, $C_2$-$C_4$-Alkenyloxy, Halogen-$C_2$-$C_4$-alkenyloxy, Phenyl-$C_2$-$C_4$-alkenyloxy, $C_2$-$C_4$-Alkinyloxy, Halogen-$C_2$-$C_4$-alkinyloxy, Phenyl-$C_2$-$C_4$-alkinyloxy, Aryloxy, Halogenaryloxy, $C_1$-$C_4$-Alkylaryloxy, Arylamino, Halogenarylamino, $C_1$-$C_4$-Alkylarylamino, Aryl-N-$C_1$-$C_4$-alkyl-amino, Aryl-N-$C_1$-$C_4$-acyl-amino, Aroyl, Halogenaroyl, $C_1$-$C_4$-Alkylaroyl, Aryl, Halogenaryl, $C_1$-$C_4$-Alkylaryl oder durch Halogen einoder mehrfach substituiertes Phenyl oder Pyridyl

darstellen.

2. Aromatische Alkanderivate der Formel I gemäß Anspruch 1, in der

$R_1$ Chlorphenyl, Bromphenyl, Fluorphenyl, Methylphenyl, Methoxyphenyl, Ethoxyphenyl, Difluormethoxyphenyl, Fluorethoxyphenyl oder Trifluorethoxyphenyl,

$R_2$ Methyl,

$R_3$ Wasserstoff und

$R_4$ Phenoxyphenyl, Fluor-phenoxyphenyl oder Phenoxypyridyl darstellen.

3. Verfahren zur Herstellung von aromatischen Alkanderivaten gemäß Ansprüchen 1 bis 2, dadurch gekennzeichnet, daß man

a) Verbindungen der allgemeinen Formel

$$R_5\overset{\oplus}{P} - CH_2\ CH_2R_4X^{\ominus} \qquad II$$

oder

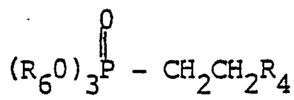

V

zunächst mit einer Base und darauf mit Verbindungen der allgemeinen Formel

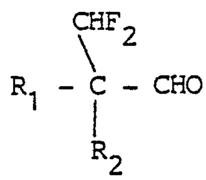

III

unter Bildung von Verbindungen der allgemeinen Formel

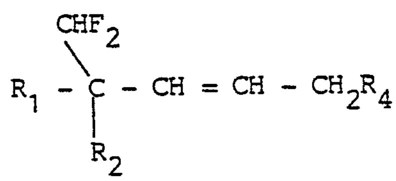

IV

und diese zu den gewünschten Verfahrensprodukten reduziert oder

b) Verbindungen der allgemeinen Formel

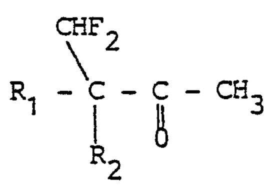

VI

mit Aldehyden der allgemeinen Formel

$R_4$ - CHO

zu α,β-ungesättigten Carbonylverbindungen der allgemeinen Formel

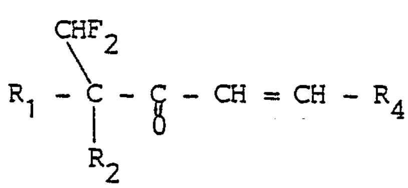

VIII

kondensiert und dann zu den gewünschten Verfahrensprodukten reduziert oder

c) Verbindungen der allgemeinen Formel

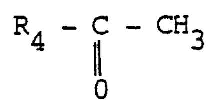

IX

mit Aldehyden der allgemeinen Formel

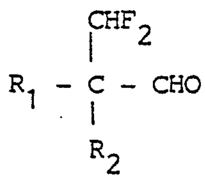

III

zu α,β-ungesättigten Carbonylverbindungen der allgemeinen Formel

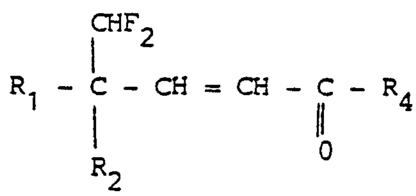

X

kondensiert und diese zu den gewünschten Verfahrensprodukten reduziert, worin $R_1$, $R_2$, $R_3$ und $R_4$ die oben genannte Bedeutung haben, $R_5$ eine Alkyloder Phenyl-Gruppe, $R_6$ eine Alkylgruppe und X ein Halogenatom darstellen.

4. Schädlingsbekämpfungsmittel, gekennzeichnet durch einen Gehalt an mindestens einer Verbindung gemäß Ansprüchen 1 und 2.

5. Schädlingsbekämpfungsmittel gemäß Anspruch 5 in Mischung mit Träger- und/oder Hilfsstoffen.

6. Verwendung von Verbindungen gemäß Ansprüchen 1 und 2 zur Bekämpfung von Schädlingen, insbesondere Insekten und Milben.

Europäisches Patentamt

# EUROPÄISCHER RECHERCHENBERICHT

Nummer der Anmeldung

EP 86730022.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A | DE - A1 - 3 317 908 (MITSUI TOATSU CHEMICALS)<br>* Ansprüche 1,3-8; Seiten 93-115 *<br>-- | 1-6 | C 07 C 43/29<br>C 07 C 143/68<br>C 07 C 147/06<br>C 07 D 213/64<br>A 01 N 31/14<br>A 01 N 41/02<br>A 01 N 43/40 |
| A | DE - A1 - 3 117 510 (MITSUI TOATSU CHEMICALS)<br>* Ansprüche 1,12,13 *<br>---- | 1,4-6 | |

RECHERCHIERTE SACHGEBIETE (Int. Cl.4)

C 07 C 43/00
C 07 C 143/00
C 07 C 147/00
C 07 D 213/00

Der vorliegende Recherchenbericht wurde für alle Patentansprüche erstellt.

| Recherchenort | Abschlußdatum der Recherche | Prüfer |
|---|---|---|
| WIEN | 21-04-1986 | REIF |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument